(19) Europäisches Patentamt
European Patent Office
Office européen des brevets

(11) Veröffentlichungsnummer : **0 440 949 B1**

# (12) EUROPÄISCHE PATENTSCHRIFT

(45) Veröffentlichungstag der Patentschrift : **27.10.93 Patentblatt 93/43**

(51) Int. Cl.[5] : **C07D 249/08,** C07D 233/60, A01N 43/653, A01N 43/50

(21) Anmeldenummer : **90124509.2**

(22) Anmeldetag : **18.12.90**

## (54) Halogenallyl-azolyl-Derivate.

Die Akte enthält technische Angaben, die nach dem Eingang der Anmeldung eingereicht wurden und die nicht in dieser Patentschrift enthalten sind.

(30) Priorität : **03.02.90 DE 4003181**

(43) Veröffentlichungstag der Anmeldung : **14.08.91 Patentblatt 91/33**

(45) Bekanntmachung des Hinweises auf die Patenterteilung : **27.10.93 Patentblatt 93/43**

(84) Benannte Vertragsstaaten : **BE CH DE FR GB IT LI NL**

(56) Entgegenhaltungen :
**EP-A- 0 097 425**
**EP-A- 0 318 400**

(73) Patentinhaber : **BAYER AG**
**D-51368 Leverkusen (DE)**

(72) Erfinder : **Jautelat, Manfred, Dr.**
**Müllersbaum 28**
**W-5093 Burscheid (DE)**
Erfinder : **Stroech, Klaus, Dr.**
**Rolsberger Strasse 22**
**W-5650 Solingen 19 (DE)**
Erfinder : **Hänssler, Gerd, Dr.**
**Am Arenzberg 58a**
**W-5090 Leverkusen 3 (DE)**
Erfinder : **Dutzmann, Stefan, Dr.**
**Kosenberg 10**
**W-4010 Hilden (DE)**
Erfinder : **Kuck, Karl-Heinz, Dr.**
**Heerstrasse 24**
**W-4018 Langenfeld (DE)**
Erfinder : **Brandes, Wilhelm, Dr.**
**Eichendorffstrasse 3**
**W-5653 Leichlingen (DE)**

EP 0 440 949 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Halogenallyl-azolyl-Derivate, mehrere Verfahren zu deren Herstellung und deren Verwendung als Mikrobizide im Pflanzenschutz und im Materialschutz.

Es ist bekannt geworden, daß bestimmte Dihalogen-allyl-triazolyl-Derivate fungizide Eigenschaften besitzen (vgl, EP-A 0 097 425). So lassen sich zum Beispiel 4-(2,4-Dichlor-phenyl)-1,2-dibrom-4-hydroxy-5-(1,2,4-tria- zol-yl)-pent-1-en und 4-(2,4-Dichlor-phenyl)-1,2-dichlor-4-hydroxy-5-(1,2,4-triazol-1-yl)-pent-1-en zur Bekämpfung von Pilzen einsetzen. Die Wirkung dieser Stoffe ist gut, läßt aber bei niedrigen Aufwandmengen in manchen Fallen zu wünschen übrig.

Es wurden nun neue Halogenallyl-azolyl-Derivate der Formel

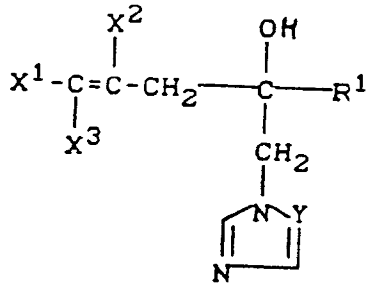

(I)

in welcher

$R^1$ für einfach bis dreifach durch Fluor, Chlor und/oder Brom substituiertes Allyl, für Propargyl oder für Cycloalkyl mit 3 bis 7 Kohlenstoffatomen steht, wobei jeder dieser Cycloalkyl-Reste einfach bis dreifach, gleichartig oder verschieden durch Alkyl mit 1 bis 4 Kohlenstoffatomen und/oder Halogen substituiert ist, oder

$R^1$ für die Reste der Formeln

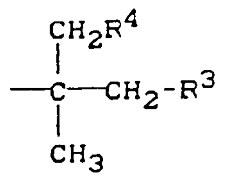

oder

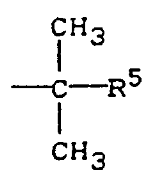

steht,

worin

$R^3$ für Wasserstoff, Fluor, Chlor, Brom, gegebenenfalls einfach bis dreifach, gleichartig oder verschieden durch Fluor, Chlor und/oder Brom substituiertes Phenyl, gegebenenfalls einfach bis dreifach, gleichartig oder verschieden durch Fluor, Chlor und/oder Brom substituiertes Phenoxy oder für gegebenenfalls einfach bis dreifach, gleichartig oder verschieden durch Fluor, Chlor und/oder Brom substituiertes Phenoxymethyl steht,

$R^4$ für Wasserstoff, Fluor, Chlor oder Brom steht

und

$R^5$ für iso-Propyl, gegebenenfalls einfach bis dreifach, gleichartig oder verschieden durch Fluor, Chlor und/oder Brom substituieres Phenyl oder für gegebenenfalls einfach bis dreifach, gleichartig oder verschieden durch Fluor, Chlor und/oder Brom substituiertes Phenoxy steht,

$X^1$ für Fluor, Chlor, Brom oder Iod steht,

$X^2$ für Fluor, Chlor, Brom oder Iod steht,

$X^3$ für Wasserstoff, Chlor, Brom oder Iod steht

und

Y für ein Stickstoffatom oder eine CH-Gruppe steht,

sowie deren Säureadditions-Salze und Metallsalz-Komplexe gefunden.

Die Verbindungen der Formel (I) enthalten ein asymmetrisch substituiertes Kohlenstoffatom und können deshalb in den beiden optischen Isomerenformen anfallen. Außerdem können die Stoffe der Formel (I) je nach der Stellung der Halogenatome an der Doppelbindung in zwei geometrischen Isomerenformen vorliegen. Die vorliegende Verbindung betrifft sowohl die Isomerengemische als auch die einzelnen Isomeren.

Weiterhin wurde gefunden, daß man Halogenallyl-azolyl-Derivate der Formel (I) sowie deren Säureadditions-Salze und Metallsalz-Komplexe erhält, wenn man

a) Alkine der Formel

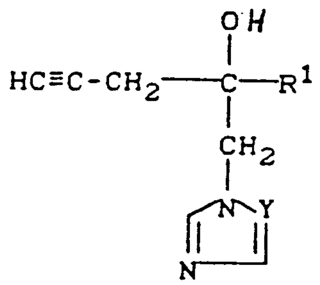

(II)

in welcher

$R^1$ und Y die oben angegebene Bedeutung haben,

mit Halogen oder Halogen liefernden Verbindungen in Gegenwart eines Verdünnungsmittels umsetzt, oder

b) Alkene der Formel

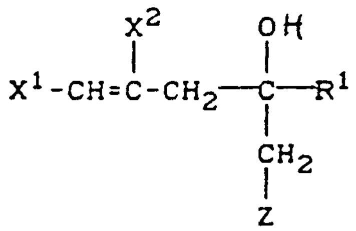

(III)

in welcher

$R^1$, $X^1$ und $X^2$ die oben angegebene Bedeutung haben und

Z für Chlor, Brom, Iod, Methylsulfonat oder p-Tolylsulfonat steht,

mit Azolen der Formel

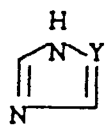

(IV)

in welcher

Y die oben angegebene Bedeutung hat, in Gegenwart eines Säurebindemittels und in Gegenwart eines Verdünnungsmittels umsetzt,

oder

c) Alkine der Formel

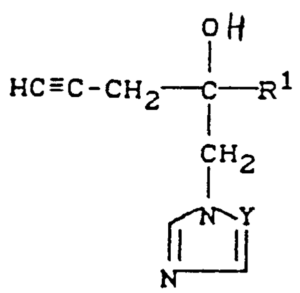

(II)

in welcher
$R^1$ und Y die oben angegebene Bedeutung haben,
in einer ersten Stufe mit Hypohalogeniten der Formel

$$MOX^4 \quad (V)$$

in welcher
M für ein Natrium- oder Kalium-Ion steht und
$X^4$ für Chlor, Brom oder Iod steht,
in Gegenwart eines Verdünnungsmittels umsetzt und in einer zweiten Stufe die so erhaltenen Halogenalkine der Formel

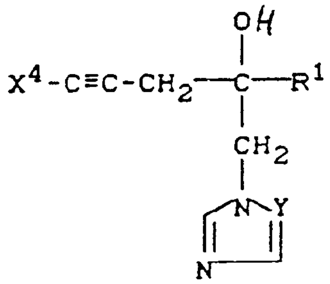

(VI)

in welcher
$R^1$, $X^4$ und Y die oben angegebene Bedeutung haben,
mit Halogen oder Halogen liefernden Verbindungen in Gegenwart eines Verdünnungsmittels umsetzt,
und gegebenenfalls anschließend an die so erhaltenen Verbindungen der Formel (I) eine Säure oder ein Metallsalz addiert.

Schließlich wurde gefunden, daß die neuen Halogenallyl-azolyl-Derivate der Formel (I) sowie deren Säureadditions-Salze und Metallsalz-Komplexe starke mikrobizide Eigenschaften besitzen und sowohl im Pflanzenschutz als auch im Materialschutz eingesetzt werden können.

Überraschenderweise besitzen die erfindungsgemäßen Stoffe sowohl im Pflanzenschutz als auch im Materialschutz eine bessere mikrobizide Wirksamkeit als die konstitutionell ähnlichsten vorbekannten Verbindungen gleicher Wirkungsrichtung.

Die erfindungsgemäßen Halogenallyl-azolyl-Derivate sind durch die Formel (I) allgemein definiert. Bevorzugt sind Verbindungen der Formel (I), in denen

$R^1$ für einfach bis dreifach durch Fluor, Chlor und/oder Brom substituiertes Allyl, für Propargyl oder für Cycloalkyl mit 3 bis 6 Kohlenstoffatomen steht, wobei jeder dieser Cycloalkyl-Reste einfach oder zweifach durch Methyl, Ethyl, Fluor, Chlor und/oder Brom substitiert ist, oder

$R^1$ für die Reste der Formeln

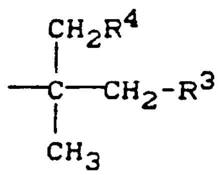

oder

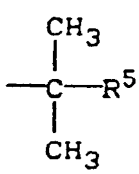

steht, worin

$R^3$ für Wasserstoff, Fluor, Chlor, gegebenenfalls einfach oder zweifach, gleichartig oder verschieden durch Fluor und/oder Chlor substituiertes Phenyl, gegebenenfalls einfach oder zweifach, gleichartig oder verschieden durch Fluor und/oder Chlor substituiertes Phenoxy oder für gegebenenfalls einfach oder zweifach, gleichartig oder verschieden durch Fluor und/oder Chlor substituiertes Phenoxymethyl steht,

$R^4$ für Wasserstoff, Fluor, Chlor steht

$R^5$ für iso-Propyl, gegebenenfalls einfach oder zweifach, gleichartig oder verschieden durch Fluor und/oder Chlor substituiertes Phenyl oder für gegebenenfalls einfach oder zweifach, gleichartig oder verschieden durch Fluor und/oder Chlor substituiertes Phenoxy steht,

$X^1$ für Fluor, Chlor, Brom oder Iod steht,

$X^2$ für Fluor, Chlor, Brom oder Iod steht,

$X^3$ für Wasserstoff, Chlor, Brom oder Iod steht und
Y für ein Stickstoffatom oder eine CH-Gruppe steht.

Bevorzugte erfindungsgemäße Verbindungen sind auch Additionspodukte aus Säuren und denjenigen Halogenallyl-azolyl-Derivaten der Formel (I), in denen $R^1$, $X^1$, $X^2$, $X^3$ und Y diejenigen Bedeutungen haben, die für diese Substituenten als bevorzugt genannt wurden.

Zu den Säuren, die addiert werden können, gehoren vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII, Nebengruppe des Periodensystems der Elemente und denjenigen Halogenallyl-azolyl-Derivaten der Formel (I), in denen $R^1$, $X^1$, $X^2$, $X^3$ und Y diejenigen Bedeutungen haben, die für diese Substituenten als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in der folgenden Tabelle aufgeführten Halogenallyl-azolyl-Derivate genannt.

<u>Tabelle 1</u>

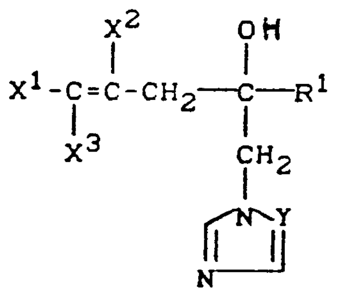

(I)

| $X^1$ | $X^2$ | $X^3$ | $R^1$ | Y |
|---|---|---|---|---|
| J | J | H | $-C(CH_3)_3$ | N |
| F | F | H | $-C(CH_3)_3$ | N |
| J | J | H | $-C(CH_3)_3$ | CH |
| F | F | H | $-C(CH_3)_3$ | CH |
| Cl | Cl | H | 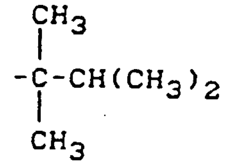 | N |
| Cl | Cl | H | 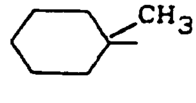 | N |

Tabelle 1 ( Fortsetzung)

| $X^1$ | $X^2$ | $X^3$ | $R^1$ | Y |
|---|---|---|---|---|
| Cl | Cl | H | 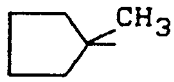 | N |
| Cl | Cl | H | 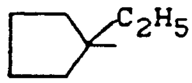 | N |
| Cl | Cl | H | 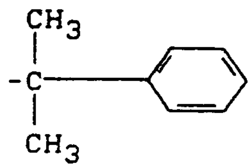 | N |
| Cl | Cl | H | 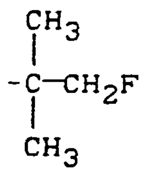 | N |
| Cl | Cl | H | 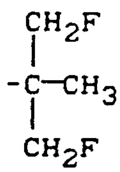 | N |
| Cl | Cl | H | 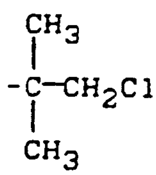 | N |
| Cl | Cl | H | 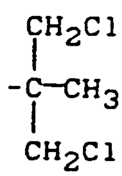 | N |

Tabelle 1 ( Fortsetzung)

| $X^1$ | $X^2$ | $X^3$ | $R^1$ | Y |
|---|---|---|---|---|
| Cl | Cl | H | 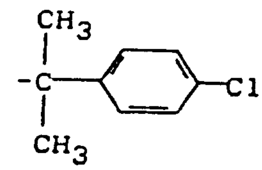 | N |
| Cl | Cl | H | 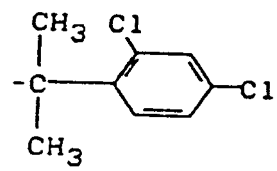 | N |
| Cl | Cl | H | 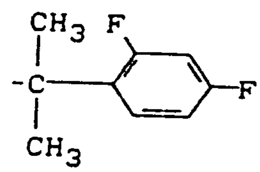 | N |
| Cl | Cl | H | 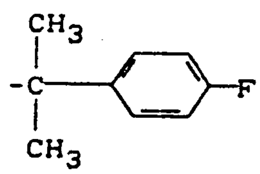 | N |
| Cl | Cl | Cl | 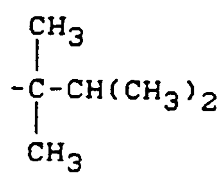 | N |
| Cl | Br | Br | 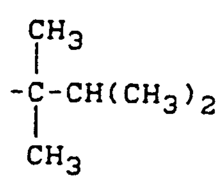 | N |
| Cl | Cl | Cl | 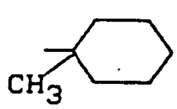 | N |
| Cl | Cl | Cl | 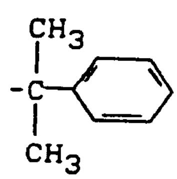 | N |
| Cl | Cl | Cl | 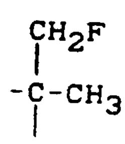 | N |

Verwendet man 4-(1-Chlor-cyclopropyl)-4-hydroxy-5-(1,2,4-triazol-1-yl)-pent-1-in als Ausgangsstoff und eine Lösung von Chlorgas in Methylenchlorid als Reaktionskomponente, so kann der Verlauf des erfindungsgemäßen Verfahrens (a) durch das folgende Formelschema veranschaulicht werden:

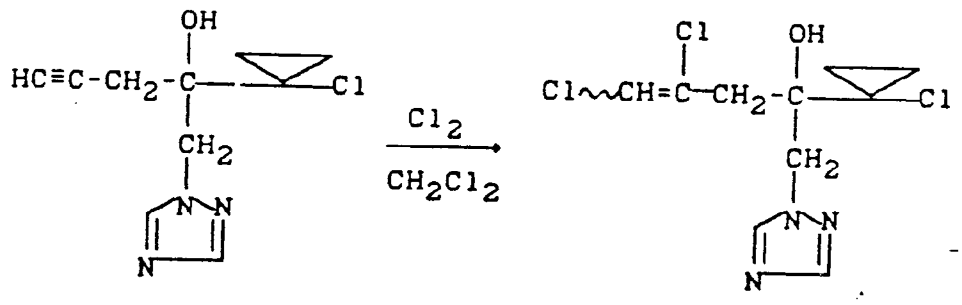


Verwendet man 1,2-Dichlor-4-hydroxy-4-chlormethyl-5,5-dimethyl-hex-1-en und 1,2,4-Triazol als Ausgangsstoffe, so kann der Verlauf des erfindungsgemäßen Verfahrens (b) durch das folgende Formelschema veranschaulicht werden:

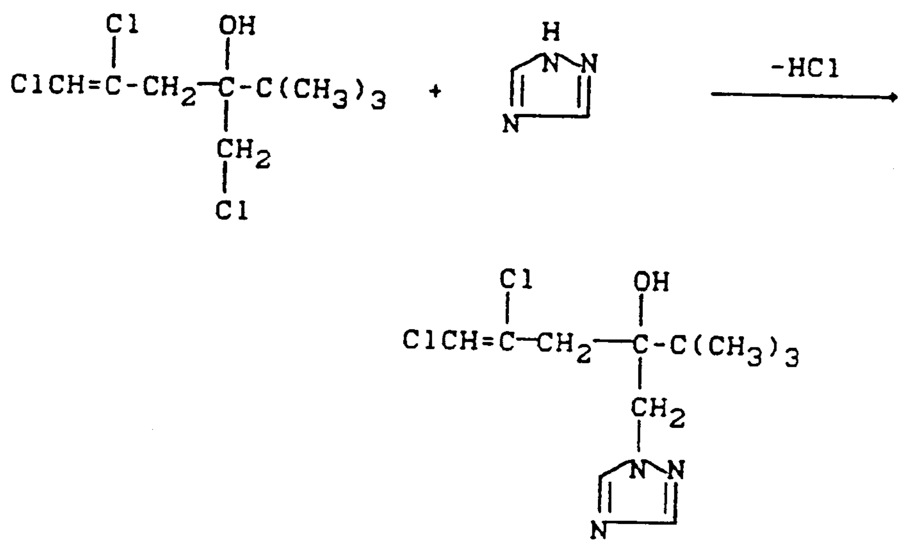


Verwendet man 4-tert.-Butyl-5-(1,2,4-triazol-1-yl)-pent-1-in-4-ol als Ausgangsstoff und Brom in Gegenwart von Kaliumhydroxid beziehungsweise Brom als Reaktionskomponenten, so kann der Verlauf des erfindungsgemäßen Verfahrens (c) durch das folgende Formelschema veranschaulicht werden:

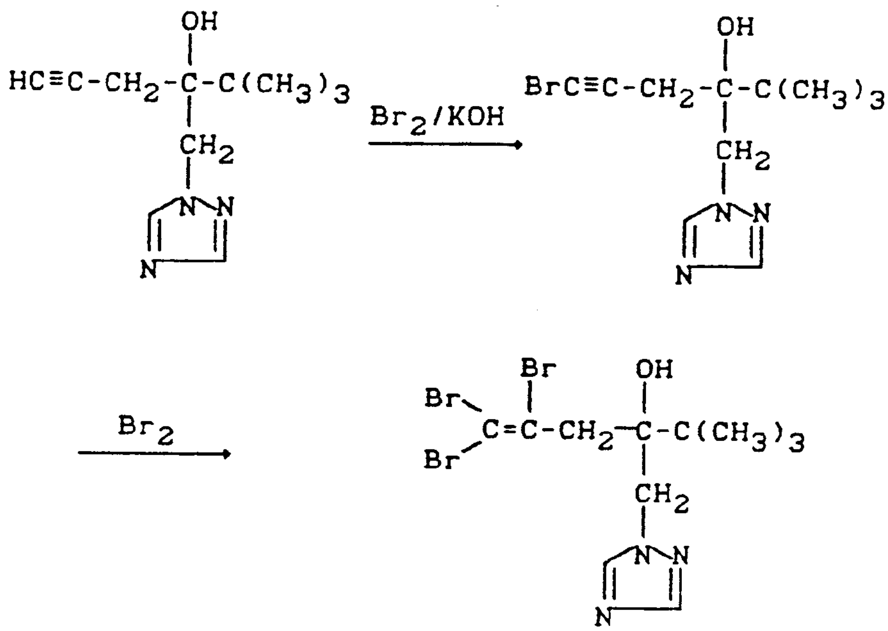


Die bei der Durchführung des erfindungsgemäßen Verfahrens (a) als Ausgangsstoffe benötigten Alkine sind durch die Formel (II) allgemein definiert. In dieser Formel haben $R^1$ und Y vorzugsweise diejenigen Bedeutungen, die bereits im Zusammenhang mit der Beschreibung der erfindungsgemäßen Stoffe der Formel (I) vorzugsweise für diese Reste genannt wurden.

Die Alkine der Formel (II) sind teilweise bekannt (vgl. EP-A 0 096 786). Sie lassen sich herstellen, indem man

d) Azolyl-methyl-ketone der Formel

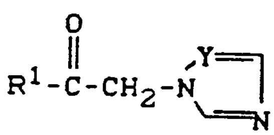 (VII)

in welcher

$R^1$ und Y die oben angegebene Bedeutung haben, mit Propargylhalogeniden der Formel

$HC{\equiv}C\text{-}CH_2\text{-}Hal$ (VIII)

in welcher

Hal für Chlor oder Brom steht,

in Gegenwart von aktiviertem Aluminium und in Gegenwart eines Verdünnungsmittels umsetzt,

oder

e) Chlormethylketone der Formel

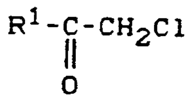 (X)

in welcher

$R^1$ die oben angegebene Bedeutung hat,

mit Propargylhalogeniden der Formel

$HC{\equiv}C\text{-}CH_2\text{-}Hal$ (VIII)

in welcher

Hal die oben angegebene Bedeutung hat,

unter den Bedingungen des Verfahrens (d) umsetzt und dann die dabei entstehenden Hydroxyalkine der

Formel

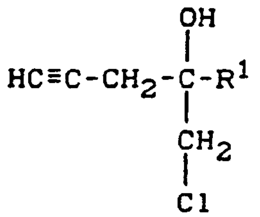

(XII)

in welcher

$R^1$ die oben angegebene Bedeutung hat,

mit Azolen der Formel

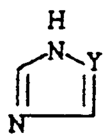

(IV)

in welcher

Y die oben angegebene Bedeutung hat,

in Gegenwart eines Säurebindemittels und in Gegenwart eines Verdünnungsmittels umsetzt.

Die bei der Durchführung des Verfahrens (d) als Ausgangsstoffe benötigten Azolyl-methyl-ketone sind durch die Formel (VII) allgemein definiert. In dieser Formel haben Y und $R^1$ vorzugsweise diejenigen Bedeutungen, die im Zusammenhang mit der Beschreibung der erfindungsgemäßen Stoffe der Formel (I) bereits vorzugsweise für diese Reste genannt wurden.

Die Azolyl-methyl-ketone der Formel (VII) sind bekannt oder lassen sich nach prinzipiell bekannten Verfahren in einfacher Weise herstellen (vgl. DE-A 2 431 407).

Die bei dem Verfahren (d) als Reaktionskomponenten benötigten Propargylhalogenide der Formel (VIII) sind bekannt.

Als Verdünnungsmittel können bei der Durchführung der ersten Stufe des Verfahrens (d) alle für derartige Umsetzungen üblichen inerten organischen Solventien verwendet werden. Vorzugsweise in Frage kommen Ether, wie Tetrahydrofuran oder Diethylether.

Bei der Durchführung des Verfahrens (d) arbeitet man in Gegenwart von aktiviertem Aluminium. Dieses wird hergestellt, indem man zu Aluminium-Schuppen katalytische Mengen an Quecksilber-(II)-chlorid und Iod zugibt.

Die Reaktionstemperaturen können bei der Durchführung des Verfahrens (d) innerhalb eines größeren Bereiches variiert werden. Im allgemeinen arbeitet man bei Temperaturen zwischen -80°C und +100°C, vorzugsweise bei Temperaturen zwischen -70°C und +60°C.

Bei der Durchführung des Verfahrens (d) arbeitet man ebenso wie bei der Durchführung der Verfahren (a), (b), (c) und (e) im allgemeinen unter Normaldruck.

Man geht bei der Durchführung des Verfahrens (d) im allgemeinen so vor, daß man auf 1 Mol an Azolyl-methyl-keton der Formel (VII) 1 bis 2 Mol Propargyl-halogenid der Formel (VIII) und 1 bis 1,5 Mol Aluminium und katalytische Mengen an Quecksilber-(II)-chlorid und Iod einsetzt. Die Isolierung der entstehenden Produkte erfolgt nach üblichen Methoden.

Die bei der Durchführung des Verfahrens (e) als Ausgangsstoffe benötigten Chlormethylketone sind durch die Formel (X) allgemein definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Chlormethylketone der Formel (X) sind bekannt oder lassen sich nach prinzipiell bekannten Methoden herstellen (vgl. DE-A 3 049 461).

Die Durchführung der ersten Stufe des Verfahrens (e) erfolgt unter den Bedingungen, die auch bei der Durchführung des Verfahrens (d) angewandt werden.

Die Hydroxyalkine der Formel (XII) können direkt weiter umgesetzt werden mit Azolen der Formel (IV). Sie können aber auch zunächst in Oxirane überführt werden und dann mit Azolen der Formel (IV) umgesetzt werden.

Bei der Durchführung der zweiten Stufe des Verfahrens (e) kommen als Säurebindemittel alle üblichen Säureakzeptoren in Frage. Vorzugsweise verwendbar sind Alkalimetallcarbonate und -hydrogencarbonate, wie Natriumcarbonat, Kaliumcarbonat oder Natriumhydrogencarbonat, ferner tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo[4.3.0]non-5-en (DBN) , 1,8-Diaza-bicyclo[5.4.0]undec-7-en (DBU) und 1,4-Diaza-bicyclo-[2.2.2]octan (DABCO).

Als Verdünnungsmittel kommen bei der Durchführung der zweiten Stufe des Verfahrens (e) alle inerten organischen Solventien in Betracht. Vorzugsweise verwendbar sind aliphatische und aromatische, gegebenenfalls halogenierte Kohlenwasserstoffe wie Pentan, Hexan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, sowie tert-Butylmethylether, Glykoldimethylether und Diglykoldimethylether, Tetrahydrofuran und Dioxan, Ketone wie Aceton, Methyl-ethyl-, Methyl-isopropyl- und Methylisobutylketon, Ester wie Essigsäuremethylester und -ethylester, Nitrile wie z.B. Acetonitril und Propionitril, und Pyridin.

Die Reaktionstemperaturen können auch bei der Durchführung der zweiten Stufe des Verfahrens (e) innerhalb eines größeren Bereiches variiert werden. Im allgemeinen arbeitet man bei Temperaturen zwischen 0°C und 200°C, vorzugsweise zwischen 20°C und 150°C.

Bei der Durchführung der zweiten Stufe des Verfahrens (e) geht man im allgemeinen so vor, daß man auf 1 Mol an Hydroxyalkin der Formel (XII) eine äquivalente Menge oder auch einen Überschuß an Azol der Formel (IV) sowie 2 bis 3 Mol an Säurebindemittel einsetzt. Die Aufarbeitung erfolgt nach üblichen Methoden. Die gegebenenfalls gewünschte weitere Umsetzung der Alkine der Formel (IIa) erfolgt bei dem Verfahren (e) in gleicher Weise wie bei dem Verfahren (d).

Als Halogene kommen bei der Durchführung des erfindungsgemäßen Verfahrens (a) vorzugsweise Fluor, Chlor, Brom und Iod als Reaktionskomponenten in Betracht, ferner gemischte Halogene wie Chlor(I)-fluorid, Brom(I)-fluorid, Iod(I)-fluorid, Brom(I)-chlorid, Jod(I)-chlorid oder Jod(I)-bromid (s. Methodicium Chimicium, F. Korte, Bd. 7, S. 842 (1976)).

Als Halogen liefernde Verbindungen können beispielsweise Sulfurylchlorid, N-Bromsuccinimid mit Salzsäure, N-Chlorsuccinimid mit Bromwasserstoffsäure oder N-Chlorsuccinimid mit Fluorwasserstoff/Pyridin (s. Synthesis 1973, 780) verwendet werden.

Die Addition der Halogene an die Alkine der Formel (II) kann durch Einwirkung von Licht, durch Wärme, durch radikalbildende Substanzen, wie organische Peroxide, durch oberflächenaktive Stoffe, wie Aktivkohle, oder Metallsalze, wie Kupfer(II)-chlorid oder Eisen(III)-chlorid, begünstigt werden. Teilweise kann dadurch das Isomerenverhältnis (E/Z) beeinflußt werden (s. Houben-Weyl, Methoden der Org. Chemie, Bd V/3, S. 551 (1962)).

Als Verdünnungsmittel können bei der Durchführung des erfindungsgemäßen Verfahrens (a) alle für derartige Umsetzungen üblichen inerten organischen Solventien eingesetzt werden. Vorzugsweise verwendbar sind halogenierte aliphatische Kohlenwasserstoffe, wie Methylenchlorid, Chloroform und Tetrachlorkohlenstoff.

Die Temperaturen können bei der Durchführung des erfindungsgemäßen Verfahrens (a) innerhalb eines bestimmten Bereiches variiert werden. Im allgemeinen arbeitet man bei Temperaturen zwischen -10°C und +120°C, vorzugsweise zwischen -5°C und +80°C.

Bei der Durchführung des erfindungsgemäßen Verfahrens (a) setzt man auf 1 Mol an Alkin der Formel (II) im allgemeinen eine äquivalente Menge oder einen Überschuß an Halogen bzw. Halogen liefernder Verbindung ein. Die Aufarbeitung erfolgt nach üblichen Methoden. Im allgemeinen geht man so vor, daß man mit einem in Wasser wenig löslichen organischen Solvens verdünnt, mit Wasser wäscht und die organische Phase nach dem Trocknen einengt. Es ist jedoch auch möglich, das Reaktionsgemisch nach beendeter Umsetzung direkt durch Abziehen der flüchtigen Komponenten unter vermindertem Druck einzuengen. Die entstehenden Produkte können gegebenenfalls nach üblichen Methoden weiter gereinigt werden.

Die bei dem erfindungsgemäßen Verfahren (b) als Ausgangsstoffe benötigten Alkene sind durch die Formel (III) allgemein definiert. In dieser Formel haben $R^1$, $X^1$ und $X^2$ vorzugsweise diejenigen Bedeutungen, die bereits im Zusammenhang mit der Beschreibung der erfindungsgemäßen Stoffe der Formel (I) vorzugsweise für diese Stoffe genannt wurden. Z steht für Chlor, Brom, Iod, Methylsulfonat oder p-Tolylsulfonat.

Die Alkene der Formel (III) lassen sich nach üblichen Methoden herstellen. So erhält man zum Beispiel Alkene der Formel (III), indem man Hydroxyalkine der Formel (XII) mit Halogenen in Gegenwart eines Verdünnungsmittels umsetzt. Die Reaktionsbedingungen entsprechen dabei denjenigen, die im Falle des erfindungsgemäßen Verfahrens (a) angewandt werden.

Als Verdünnungsmittel kommen bei der Durchführung des erfindungsgemäßen Verfahrens (b) alle üblichen inerten organischen Solventien in Betracht. Vorzugsweise verwendbar sind diejenigen Lösungsmittel, die

bereits im Zusammenhang mit der Beschreibung der zweiten Stufe des Verfahrens (e) als bevorzugte Solventien genannt wurden.

Als Säurebindemittel kommen bei der Durchführung des erfindungsgemäßen Verfahrens (b) alle üblichen Säureakzeptoren in Betracht. Vorzugsweise verwendbar sind. alle diejenigen Säurebindemittel, die bereits im Zusammenhang mit der Beschreibung der zweiten Stufe des Verfahrens (e) als bevorzugte Säureakzeptoren genannt wurden.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0°C und 150°C, vorzugsweise zwischen 20°C und 120°C.

Bei der Durchführung des erfindungsgemäßen Verfahrens (b) geht man im allgemeinen so vor, daß man auf 1 Mol an Alken der Formel (III) eine äquivalente Menge oder einen Überschuß an Azol der Formel (IV) sowie 2 bis 3 Mol an Säurebindemittel einsetzt. Die Aufarbeitung erfolgt nach üblichen Methoden.

Die bei der Durchführung des erfindungsgemäßen Verfahrens (c) als Reaktionskomponenten benötigten Hypohalogenite der Formel (V) für werden vorzugsweise aus Base und Halogen frisch zubereitet.

Als Verdünnungsmittel können bei der Durchführung des erfindungsgemäßen Verfahrens (c) sowohl bei der Durchführung der ersten als auch der zweiten Stufe alle für derartige Umsetzungen üblichen inerten organischen Solventien eingesetzt werden. Vorzugsweise verwendbar sind halogenierte aliphatische Kohlenwasserstoffe, wie Methylenchlorid, Chloroform und Tetrachlorkohlenstoff.

Die Temperaturen können bei der Durchführung des erfindungsgemäßen Verfahrens (c) sowohl in der ersten als auch in der zweiten Stufe innerhalb eines bestimmten Bereiches variiert werden. Im allgemeinen arbeitet man bei Temperaturen zwischen -10°C und +120°C, vorzugsweise zwischen -5°C und +80°C.

Bei der Durchführung der ersten Stufe des erfindungsgemäßen Verfahrens (c) setzt man auf 1 Mol an Alkin der Formel (II) im allgemeinen einen Überschuß an Hypohalogenit ein. Bei der Durchführung der zweiten Stufe des erfindungsgemäßen Verfahrens (c) setzt man auf 1 Mol an Halogenalkin der Formel (VI) im allgemeinen eine äquivalente Menge oder einen Überschuß an Halogen ein. Die Aufarbeitung erfolgt sowohl bei der Durchführung der ersten als auch der zweiten Stufe nach üblichen Methoden.

Die nach den erfindungsgemäßen Verfahren erhältlichen Halogenallyl-azolyl-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli
oder Pseudoperonospora cubensis;
Plasmopara-Arten, wie Plasmopara viticola;

Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Pseudocercosporella, Erysiphe, Fusarium, Pyrenophora, Cochliobolus, Pyricularia und Pellicularia, sowie zur Bekämpfung von Gurkenmehltau und Apfelschorf und außerdem zur Bekämpfung von Botrytis im Obst-, Wein- und Gemüsebau. Sie besitzen außerdem eine gute und breite in - vitro - Wirkung und eignen sich zur Bekämpfung echter Mehltaupilze, wie Rhizoctonia solani.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aurebasidium, wie Aureobasidium pullulans,
Sclerophoma, wie Sclerophoma pityophila,
Trichoderma, wie Trichoderma viride,
Escherichia, wie Escherichia coli,

Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Pflanzenschutz im allgemeinen zwischen 0,1 und 95 Gewichtsprozent Wirkstoff, vorzugsweise zwischen 0,5 und 90 %.

Die erfindungsgemäßen Wirkstoffe können bei Verwendung im Pflanzenschutz in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren. Die Wirkstoffe können bei Verwendung im Pflanzenschutz als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Beim Einsatz im Materialschutz richten sich die Anwendungskonzentrationen an erfindungsgemäßen Wirkstoffen nach der Art und dem Vorkmmen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Auch beim Einsatz im Materialschutz können die erfindungsgemäßen Wirkstoffe in Mischung mit anderen bekannten Wirkstoffen angewendet werden.

Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate wie 2-Phenylphenol, (2,2'-Dihrdroxy-5,5'-dichlor)-diphenylmethan und 3-Methyl-4-chlor-phenol, Organo-Zinnverbindungen, N-Trihalogenmethylthio-Verbindungen, wie Folpet, Fluorfolpet, Dichlofluanid.

Die Herstellung und die Verwendung der erfindungsgemäßen Wirkstoffe werden durch die folgenden Beispiele veranschaulicht.

## Herstellungsbeispiele

### Beispiel 1

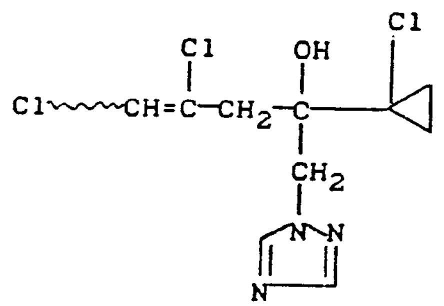

(I-1)

x HCl

225,5 g (1 Mol) 4-(1-Chlorcyclopropyl)-4-hydroxy-5-(1,2,4-triazol-l-yl)-pent-1-in in 1,8 Litern absolutem Methylenchlorid werden bei 0°C zunächst mit 5 ml einer 1 N-Lösung von Chlorwasserstoff in Ether und dann mit 1,05 Litern einer 1 N-Lösung von Chlor in Methylenchlorid versetzt. Nach 4-stündigem Rühren bei 20°C werden noch 0,1 Mol einer Lösung von Chlor hinzugefügt. Nach weiterem 2-stündigen Rühren tropft man unter Kühlung 360 ml (1 Mol) einer Lösung von Chlorwasserstoff in Ether zu, wobei ein Salz ausfällt.

Das Reaktionsgemisch wird 16 Stunden bei Raumtemperatur stehen gelassen und dann abgesaugt. Die verbleibende organische Phase wird durch Abziehen des Lösungsmittels unter vermindertem Druck eingeengt. Man erhält auf diese Weise 210 g (0,63 Mol, 63 %) 4-(1-Chlorcyclopropyl)-1,2 dichlor-4-hydroxy-5-(1,2,4-triazol-1-yl)-pent-1-en- hydrochlorid in Form eines Feststoffes vom Schmelzpunkt 171-173° C.

NMR ($CDCl_3$): δ=0,6 (m, 2H); 0,9 (m, 1H); 1,25 (m, 1H); 3,2 (AB, 2H); 4,9 (AB, 2H); 6,4 (s, 1H), 6,7 (OH und NH); 8,45 (s, 1H); 9,8 (s, 1H)

### Beispiel 2

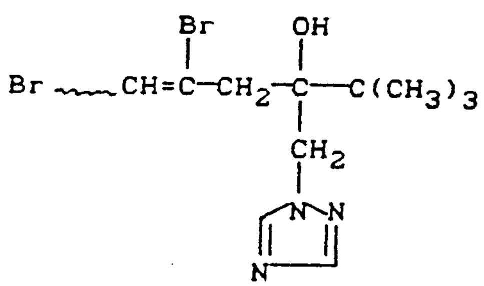

(I-2)

In eine Lösung von 5 g (24 mMol) 4-tert.-Butyl-5-(1,2,4-triazol-1-yl)-pent-1-in-4-ol in 30 ml Dichlormethan werden bei 0°C unter Rühren 2,4 g (24 mMol) konzentrierte Schwefelsäure langsam eingetropft. Danach tropft man unter Belichtung und unter Rühren eine Lösung von 4 g (25 mMol) Brom in 50 ml Dichlormethan hinzu. Man läßt die Temperatur des Reaktionsgemisches auf Raumtemperatur ansteigen und rührt noch eine Stunde bei Raumtemperatur. Das Reaktionsgemisch wird je zweimal mit wäßriger Natriumcarbonat-Lösung und mit

Wasser ausgeschüttelt, dann über Natriumsulfat getrocknet und durch Abziehen der flüchtigen Anteile unter vermindertem Druck eingeengt. Man erhält auf diese Weise 7,7 g (87 % der Theorie) 4-t-Butyl-1,2-dibrom-5-(1,2,4-triazol-1-yl)-pent-1-en-4-ol in Form eines Feststoffes mit dem Schmelzpunkt 81°C.

Beispiel 3

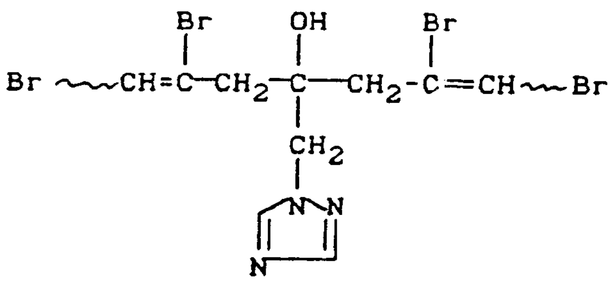

(I-3)

In eine Lösung von 5 g (26 mMol) 4-(1,2,4-Triazol-1-yl-methyl)-hepta-1,6-dien-4-ol in 50 ml Dichlormethan werden bei 0°C unter Rühren langsam 2,55 g (26 mMol) konzentrierte Schwefelsäure eingetropft. Danach tropft man unter Belichtung und unter Rühren bei der gleichen Temperatur eine Lösung von 8,5 g (53 mMol) Brom in 20 ml Dichlormethan hinzu. Man läßt die Temperatur des Reaktionsgemisches auf Raumtemperatur ansteigen und rührt noch eine Stunde bei Raumtemperatur nach. Das Reaktionsgemisch wird je zweimal mit wäßriger Natriumcarbonat-Lösung und mit Wasser ausgeschüttelt, dann über Natriumsulfat getrocknet und unter vermindertem Druck eingedampft. Der verbleibende Rückstand wird mit einem Gemisch aus Cyclohexan/Dichlormethan = 1:1 als Laufmittel an Kieselgel chromatographiert. Man erhält auf diese Weise 7,8 g (58 % der Theorie) 1,2,6,7-Tetrabrom-4-(1,2,4-triazol-1-yl-methyl)-hepta-1,6-dien-4-ol in Form eines Feststoffes mit dem Schmelzpunkt 100°C.

Beispiel 4

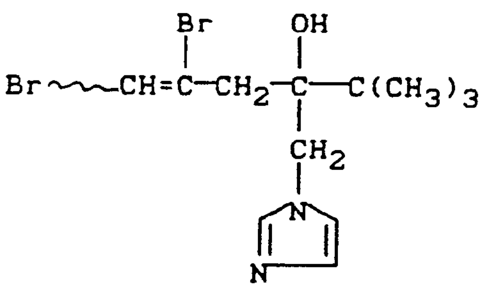

(I-4)

In eine Lösung von 3 g (14,6 mMol) 4-tert.-Butyl-5-(imidazol-1-yl)-pent-1-in-4-ol in 20 ml Dichlormethan werden bei 0°C unter Rühren 1,5 g (15 mMol) konzentrierte Schwefelsäure langsam eingetropft. Danach tropft man unter Belichtung und unter Rühren eine Lösung von 2,5 g (15,6 mMol) Brom in 20 ml Dichlormethan hinzu. Man läßt die Temperatur des Reaktionsgemisches auf Raumtemperatur ansteigen und rührt noch eine Stunde bei Raumtemperatur. Das Reaktionsgemisch wird je zweimal mit wäßriger Natriumcarbonat-Lösung und mit Wasser ausgeschüttelt, dann über Natriumsulfat getrocknet und unter vermindertem Druck eingeengt. Man erhält auf diese Weise 4,2 g (77 % der Theorie) 4-t-Butyl-1,2-dibrom-5-(imidazol-1-yl )pent-1-en-4-ol.

$^1$H-NMR (200 MHz, $CDCl_3$):

δ = 1,02(s, 9H), 2,79 (d, 1H), 3,14 (d, 1H), 4,07 (d, 1H), 4,22 (d, 1H), 6,61 (s, 1H), 7,03-7,13 (m, 2H), 7,67 (s,1 H) ppm.

Beispiel 5

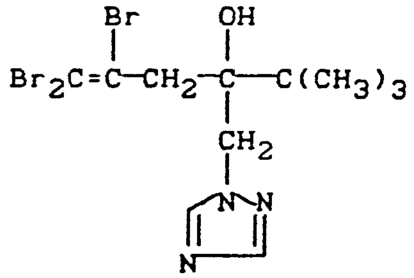

(I-5)

In eine Lösung von 2 g (7 mMol) 1-Brom-4-tert.-butyl-5-(1,2,4-triazol-1-yl)-pent-1-in-4-ol in 20 ml Dichlormethan werden bei 0°C unter Rühren langsam 0,7 g (7 mMol) konzentrierte Schwefelsäure eingetropft. Danach tropft man bei der gleichen Temperatur unter Belichtung und unter Rühren eine Lösung von 1,3 g (8 mMol) Brom in 10 ml Dichlormethan hinzu. Man läßt die Temperatur des Reaktionsgemisches auf Raumtemperatur ansteigen und rührt noch zwei Stunden bei Raumtemperatur nach. Das Reaktionsgemisch wird durch Zugabe wäßriger Natriumcarbonat-Lösung basisch gestellt. Die organische Phase wird abgetrennt, mit Wasser gewaschen, über Natriumsulfat getrocknet und unter vermindertem Druck eingeengt. Man erhält auf diese Weise 3,1 g (99 % der Theorie) 4-t-Butyl-5-(1,2,4-triazol-1-yl)-1,1,2-tribrompent-1-en-4-ol in Form eines öligen Produktes.

$^1$H-NMR (200 MHz, $CDCl_3$):

δ = 0,96(s, 9H), 2,87 (d, 1H), 3,43 (d, 1H), 4,3 (breit), 4,46 (s, 2H), 8,0 (s, 1H), 8,28 (s, 1H) ppm.

Herstellung der Ausgangsverbindung

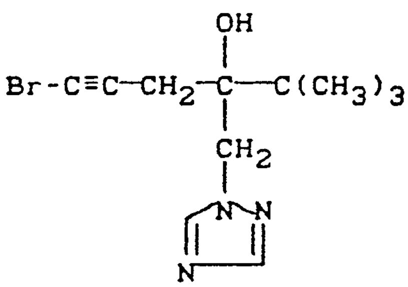

(II-1)

Unter Rühren werden 7,7 g (48 mMol) Brom bei Temperaturen zwischen 0°C und 5°C in eine Lösung vom 7,2 g (0,13 Mol) Kaliumhydroxid in 20 ml Wasser eingetropft. Man überführt diese Lösung in einen Tropftrichter und tropft sie unter Stickstoffatmosphäre und unter Rühren bei Raumtemperatur in eine Lösung von 5 g (24 mMol) 4-tert.-Butyl-5-(1,2,4-triazol-1-yl)-pent-1-in-4-ol in 20 ml Tetrahydrofuran. Man läßt 3 Stunden bei 25 bis 30°C nachreagieren und gießt das Reaktionsgemisch anschließend auf Eiswasser. Das entstehende Gemisch wird mit Essigsäureethylester extrahiert. Die vereinigten organischen Phasen werden über Natriumsulfat getrocknet und-durch Abziehen des Lösungsmittels unter vermindertem Druck eingeengt. Man erhält auf diese Weise 6,3 g (91 % der Theorie) 1-Brom-4-t-butyl-5-(1,2,4-triazol-1-yl)-pent-1-in-4-ol in Form eines öligen Produktes.

$^1$H-NMR (200 MHz, $CDCl_3$):

δ = 1,08 (s, 9H), 2,13 (d, 1H), 2,57 (d, 1H), 3,78 (breit, 1H), 4,33 (d, 1H), 4,58 (d, 1H), 8,02 (s, 1H), 8,44 (s, 1H) ppm.

Nach den zuvor angegebenen Methoden werden auch die in der folgenden Tabelle 2 aufgeführten Stoffe der Formel

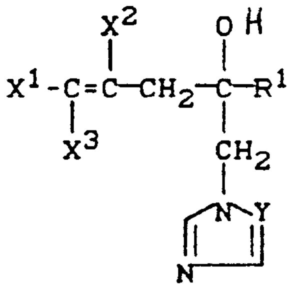

(I)

hergestellt.

<u>Tabelle 2</u>

| Beispiele Nr. | Verb. Nr. | $X^1$ | $X^2$ | $X^3$ | $R^1$ | Y | Schmelzpunkt (°C) oder $^1H$-NMR ($CDCl_3$, 200 MHz) |
|---|---|---|---|---|---|---|---|
| 6 | I-6 | Br | Br | Br | 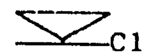 | N | δ = 0,34-0,57 (m, 2H), 0,77-0,9 (m, 1H), 1,02-1,18 (m, 1H), 3,21 (d, 1H), 3,59 (d, 1H), 4,48 (breit) und 4,48 (d, 2H), 4,95 (d, 1H), 8,02 (2, 1H), 8,39 (s, 1H) ppm |
| 7 | I-7 | Br | Br | H | 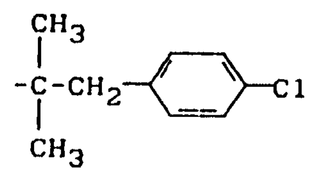 | N | δ = 0,83 (s, 3H), 0,88 (s, 3H), 2,65 (d, 1H), 2,75 (d, 1H), 2,92 (d, 1H), 3,32 (d, 1H), 4,49 (d, 1H), 4,52 (d, 1H), 6,57 (s, 1H), 7,07 (d, 2H), 7,28 (d, 2H), 8,0 (s, 1H), 8,26 ((s, 1H)ppm |

Tabelle 2 (Fortsetzung)

| Beispiele Nr. | Verb. Nr. | $X^1$ | $X^2$ | $X^3$ | $R^1$ | Y | Schmelzpunkt (°C) oder $^1H$-NMR ($CDCl_3$, 200 MHz) |
|---|---|---|---|---|---|---|---|
| 8 | I-8 | Br | Br | H | 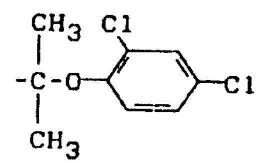 | N | δ = 1,25 (s, 3H), 1,32 (s, 3H), 3,2 (d, 1H), 3,4 (d, 1H), 4,66 (d, 1H), 4,75 (d, 1H), 6,65 (s, 1H), 7,05 (d, 1H), 7,17 (dd, 1H), 7,4 (d, 1H), 7,93 (s, 1H), 8,35 (s, 1H) ppm |
| 9 | I-9 | Br | Br | H | 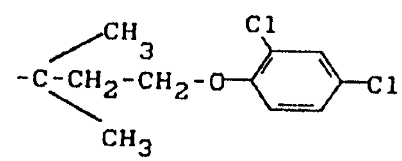 | N | δ = 1,02 (s, 3H) 1,12 (s, 3H), 2,0 (m, 2H), 2,86 (d, 1H), 3,32 (d, 1H), 4,15 (m, 2H), 4,48 (s, 2H), 6,53 (s, 1H), 6,87 (d, 1H), 7,18 (dd, 1H), 7,38 (d, 1H), 7,98 (s, 1H), 8,22 ((s, 1H)ppm |

Tabelle 2 (Fortsetzung)

| Beispiele Nr. | Verb. Nr. | $X^1$ | $X^2$ | $X^3$ | $R^1$ | Y | Schmelzpunkt (°C) oder $^1H$-NMR ($CDCl_3$, 200 MHz) |
|---|---|---|---|---|---|---|---|
| 10 | I-10 | Br | Br | H | 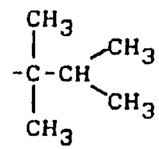 | N | δ = 0,78 (s, 3H), 0,95 (d, 3H), 0,97 (s, 3H), 1,04 (d, 3H), 1,87 (hept, 1H), 2,87 (d, 1H), 3,29 (d, 1H), 4,28 (s, 1H), 4,41 (d, 1H), 4,51 (d, 1H), 6,52 (s, 1H), 7,96 (s, 1H), 8,21 (s, 1H) ppm. |
| 11 | I-11 | Br | Br | H | 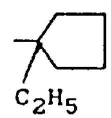 | N | δ = 0,95 (t, 3H) 1,4 - 2,0 (m, 8H), 2,86 (d, 1H), 3,27 (d, 1H), 4,25 (1H), 4,48 (s, 1H), 4,49 (s, 1H), 6,54 (s, 1H), 8,05 (s, 1H), 8,65 (s, 1H) ppm. |

<u>Tabelle 2</u> (Fortsetzung)

| Beispiele Nr. | Verb. Nr. | $X^1$ | $X^2$ | $X^3$ | $R^1$ | Y | Schmelzpunkt (°C) oder $^1H$-NMR ($CDCl_3$, 200 MHz) |
|---|---|---|---|---|---|---|---|
| 12 | I-12 | Br | Br | H | 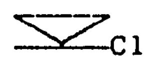 | N | δ = 0,32-0,54 (m, 2H), 0,78-0,90 (m, 1H), 1,03-1,17 (m, 1H), 3,06 (d, 1H), 3,55 (m, 1H), 4,44 (d, 1H), 4,93 (d, 1H), 6,87 (s, 1H), 8,04 (s, 1H), 8,32 (s, 1H) ppm. |
| 13 | I-13 | Br | Br | H | 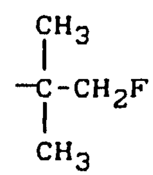 | N | δ = 0,91-2,00 (m, 6H), 2,83 (d, 1H), 3,26 (d, 1H), 4,2-5,2 (m, 5H), 6,54 (s, 1H), 7,96 (s, 1H), 8,22 (s, 1H) ppm. |

Tabelle 2 (Fortsetzung)

| Beispiele Nr. | Verb. Nr. | $X^1$ | $X^2$ | $X^3$ | $R^1$ | Y | Schmelzpunkt (°C) oder $^1$H-NMR ($CDCl_3$, 200 MHz) |
|---|---|---|---|---|---|---|---|
| 14 | I-14 | Br | Br | H | 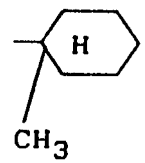 | N | δ = 1,04 (s, 3H), 1,1-1,7 (m, 10H), 2,82 (d, 1H), 3,22 (d, 1H), 4,41 (d, 1H), 4,51 (d, 1H), 6,52 (s, 1H), 8,02 (s, 1H), 8,33 (s, 1H) ppm. |
| 15 | I-15 | Br | Br | H | 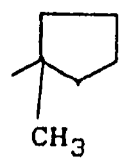 | N | 120° C |
| 16 | I-16 | Cl | Cl | H | $-C(CH_3)_3$ | N | δ = 6,2 (s, -CCl=CHCl) |
| 17 | I-17 | Cl | Cl | H | 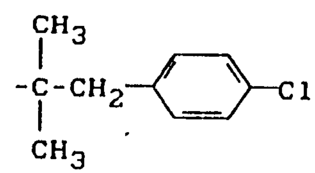 | N | δ = 6,25 (s, -CCl=CHCl) |

<u>Tabelle 2</u> (Fortsetzung)

| Beispiele Nr. | Verb. Nr. | $X^1$ | $X^2$ | $X^3$ | $R^1$ | Y | Schmelzpunkt (°C) oder $^1H$-NMR ($CDCl_3$, 200 MHz) |
|---|---|---|---|---|---|---|---|
| 18 | I-18 | Cl | Cl | H | 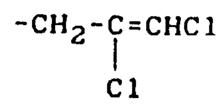 | N | $\delta$ = 6,45 (s, -CCl=CHCl) |
| 19 | I-19 | Cl | Cl | H | $-CH_2-C\equiv CH$ | N | $\delta$ = 6,4 (s, -CCl=CHCl) |
| 20 | I-20 | Cl | Cl | H | 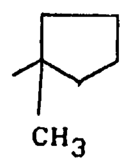 | N | Fp. 88-89° |
| 21 | I-21 | Cl | Cl | H | 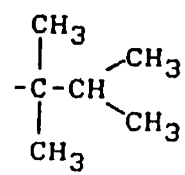 | N | (x HCl) Fp. 49-52° |
| 22 | I-22 | Cl | Cl | H | 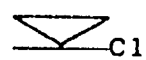 | N | $\delta$ = 6,45 (s, -CCl=CHCl) |

In den folgenden Verwendungsbeispielen wurden die Verbindungen der nachstehend aufgeführten For-

meln als Vergleichssubstanzen eingesetzt:

(A) = 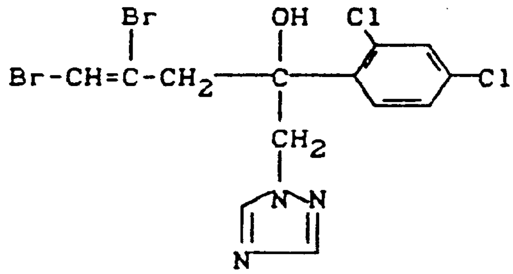

(B) = 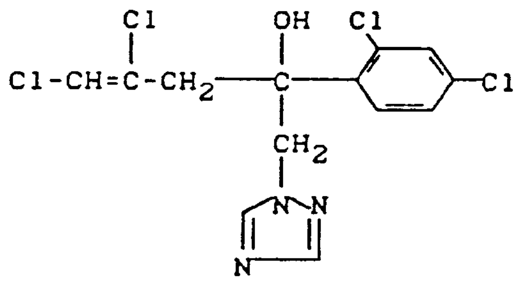

(Bekannt aus EP-A 0 097 425)

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht, Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Stoffe (I-6), (I-10) und (I-13) eine sehr gute Wirksamkeit.

Beispiel B

Erysiphe-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-10) eine sehr gute Wirksamkeit.

Beispiel C

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100% relativer Luffeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

In diesem Test zeigt die erfindungsgemäße Verbindung (I-13) eine sehr gute Wirksamkeit.

Beispiel D

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % realtiver Luftfeuchtugkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

In diesem Test zeigen die erfindungsgemäßen Verbindungen (I-6) und (I-10) eine sehr gute Wirksamkeit.

Beispiel E

Fusarium nivale-Test (Roggen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10°C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome des Schneeschimmels.

In diesem Test zeigen die erfindungsgemäßen Verbindungen (I-1), (I-2), (I-3), (I-5), (I-6), (I-8), (I-10), (I-12), (I-13) und (I-21) eine bessere Wirksamkeit als die Vergleichssubstanz (A).

Beispiel F

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Den Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (I-1), (I-3), (I-6), (I-12) und (I-13) eine bessere Wirksamkeit als die Vergleichssubstanz (B).

Beispiel G

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man Junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalles.

In diesem Test zeigen die erfindungsgemäßen Stoffe (I-2), (I-6), (I-12), (I-21) und (I-22) eine bessere Wirksamkeit als die Vergleichssubstanz (A).

Beispiel H

Pyricularia-Test (Reis)/systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer relativen Luftfeuchtigkeit von 100 % bis zur Auswertung.

4 Trage nach der Inokulation erfolgt die Auswertung des Krankheitsbefalles.

In diesem Test zeigen die erfindungsgemäßen Verbindungen (I-2) und (I-22) eine bessere Wirksamkeit als die Vergleichssubstanz (B).

Beispiel I

Pellicularia-Test (Reis) / protektiv

Lösungsmittel: 12,5 Gewichtsteile Aceton

Emulgator: 0,3 Gewichtsteile Alkylarylpolyglykol ether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

In diesem Test zeigen die erfindungsgemäßen Stoffe (I-2), (I-6), (I-12), (I-21) und (I-22) eine bessere Wirksamkeit als die Vergleichssubstanz (B).

Beispiel K

Fusarium culmorum-Test / Weizen / protektiv

Lösungsmittel: 100 Gewichtsteile Aceton
Emulgator: 4,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man 7 Tage alte Pflanzen mit der Wirkstoffzubereitung bis zur Tropfnässe. Nach 24 Stunden werden die Pflanzen mit Konidien von Fusarium culmorum, welche in einer 2,4 %igen Potato-Dextrose-Broth (Difco) suspendiert sind, besprüht, Unmittelbar vorher werden die Blätter durch Nadelstiche verletzt. Die Pflanzen verbleiben anschließend bis zur Auswertung 7 Tage in einer lichtdurchlässigen Inkubationskammer, in der die Temperatur tagsüber 27°C und nachts 21°C beträgt und in der eine Luftfeuchte von 100 % herrscht.

In diesem Test zeigen die erfindungsgemäßen Verbindungen (I-2), (I-5), (I-6), (I-7), (I-10), (I-11), (I-12) und (I-13) eine sehr gute Wirksamkeit.

Beispiel L

Gibberella zeae (=Fusarium graminearum)-Test /Gerste/ protektiv

Lösungsmittel: 100 Gewichtsteile Aceton
Emulgator: 4,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man ca. 7 Tage alte Pflanzen mit der Wirkstoffzubereitung bis zur Tropfnässe. Nach 24 Stunden werden die Pflanzen mit Konidien von Gibberella zeae, welche in einer 2,4 %igen Potato-Dextrose-Broth (Difco) suspendiert sind, besprüht. Unmittelbar vorher werden die Blätter durch Nadelstiche verletzt. Die Pflanzen verbleiben anschließend bis zur Auswertung 7 Tage in einer lichtdurchlässigen Inkubationskammer, in der die Temperatur tagsüber 27°C und nachts 21°C beträgt und in der eine Luftfeuchte von 100 % herrscht.

In diesem Test zeigen die erfindungsgemäßen Verbindungen (I-2), (I-5), (I-6), (I-7), (I-10), (I-11), (I-12), (I-13) und (I-16) eine sehr gute Wirksamkeit.

Beispiel M

Fusarium nivale-Test / Weizen / protektiv

Lösungsmittel: 100 Gewichtsteile Aceton
Emulgator: 4,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man ca. 7 Tage alte Pflanzen mit der Wirkstoffzubereitung bis zur Tropfnässe. Nach 24 Stunden werden die Pflanzen mit Konidien von Fusarium nivale, welche in

einer 2,4 %igen Potato-Dextrose-Broth (Difco) suspendiert sind, besprüht. Unmittelbar vorher werden die Blätter durch Nadelstiche verletzt. Die Pflanzen verbleiben anschließend bis zur Auswertung 5-6 Tage in einer lichtdurchlässigen Inkubationskammer, in der die Temperatur tagsüber 20°C und nachts 15°C beträgt und in der eine Luftfeuchte von 100 % herrscht.

In diesem Test zeige die erfindungsgemäßen Verbindungen (I-2), (I-5), (I-6), (I-7), (I-10), (I-11), (I-12) und (I-13) eine sehr gute Wirksamkeit.

Beispiel N

Pseudocercosporella herpotrichoides-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung.

In diesem Test zeigen die erfindungsgemäßen Stoffe (I-1), (I-2) und (I-10) eine bessere Wirksamkeit als die Vergleichssubstanzen (A) und (B).

Beispiel O

Botrytis-Test (Bohne) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

In diesem Test zeigen die erfindungsgemäßen Verbindungen (I-1), (I-2), (I-6), (I-7), (I-10) und (I-12) eine sehr gute Wirksamkeit.

Beispiel P

Venturia-Test (Apfel) / protektiv

Lösungsmittel: 4,7 Gewichtsteile Aceton
Emulgator: 0,3 Gewichtsteile Alkylarylpoly glykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigen die erfindungsgemäßen Verbindungen (I-5), (I-6), (I-7), (I-9), (I-10) und (I-16) eine sehr gute Wirksamkeit.

## Patentansprüche

1. Halogenallyl-azolyl-Derivate der Formel

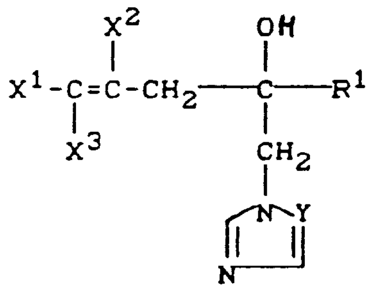

(I)

in welcher

$R^1$ für einfach bis dreifach durch Fluor, Chlor und/oder Brom substituiertes Allyl, für Propargyl oder für Cycloalkyl mit 3 bis 7 Kohlenstoffatomen steht, wobei jeder dieser Cycloalkyl-Reste einfach bis dreifach, gleichartig oder verschieden durch Alkyl mit 1 bis 4 Kohlenstoffatomen und/oder Halogen substituiert ist, oder

$R^1$ für die Reste der Formeln

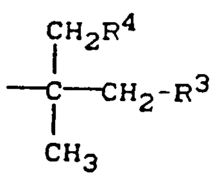

oder

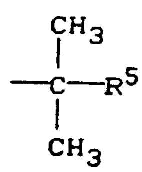

steht,

worin

$R^3$ für Wasserstoff, Fluor, Chlor, Brom, gegebenenfalls einfach bis dreifach, gleichartig oder verschieden durch Fluor, Chlor und/oder Brom substituiertes Phenyl, gegebenenfalls einfach bis dreifach, gleichartig oder verschieden durch Fluor, Chlor und/oder Brom substituiertes Phenoxy oder für gegebenenfalls einfach bis dreifach, gleichartig oder verschieden durch Fluor, Chlor und/oder Brom substituiertes Phenoxymethyl steht,

$R^4$ für Wasserstoff, Fluor, Chlor oder Brom steht

und

$R^5$ für iso-Propyl, gegebenenfalls einfach bis dreifach, gleichartig oder verschieden durch Fluor, Chlor und/oder Brom substituieres Phenyl oder für gegebenenfalls einfach bis dreifach, gleichartig oder verschieden durch Fluor, Chlor und/oder Brom substituiertes Phenoxy steht,

$X^1$ für Fluor, Chlor, Brom oder Iod steht,

$X^2$ für Fluor, Chlor, Brom oder Iod steht,

$X^3$ für Wasserstoff, Chlor, Brom oder Iod steht

und

Y für ein Stickstoffatom oder eine CH-Gruppe steht),

sowie deren Säureadditions-Salze und Metallsalz-Komplexe.

2. Verfahren zur Herstellung von Halogenallyl-azolyl-Derivaten der Formel (I) gemäß Anspruch 1 sowie von deren Säureadditions-Salzen und Metallsalz-Komplexen,
dadurch gekennzeichnet, daß man
   a) Alkine der Formel

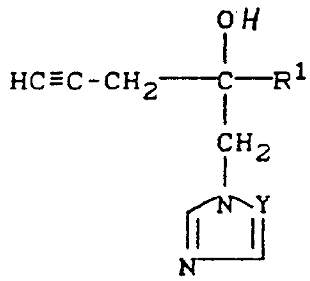

(II)

in welcher

R¹ und Y die oben angegebene Bedeutung haben,

mit Halogen oder Halogen liefernden Verbindungen in Gegenwart eines Verdünnungsmittels umsetzt, oder

b) Alkene der Formel

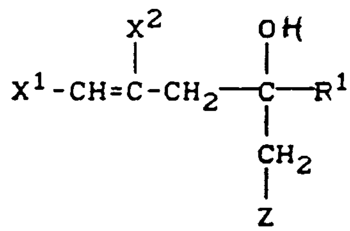

(III)

in welcher

$R^1$, $X^1$ und $X^2$ die oben angegebene Bedeutung haben und

Z für Chlor, Brom, Iod, Methylsulfonat oder p-Tolylsulfonat steht,

mit Azolen der Formel

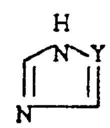

(IV)

in welcher

Y die oben angegebene Bedeutung hat,

in Gegenwart eines Säurebindemittels und in Gegenwart eines Verdünnungsmittels umsetzt, oder

c) Alkine der Formel

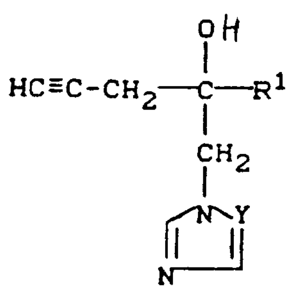

(II)

in welcher

$R^1$ und Y die oben angegebene Bedeutung haben, in einer ersten Stufe mit Hypohalogeniten der Formel

$$MOX^4 \quad (V)$$

in welcher

M für ein Natrium- oder Kalium-Ion steht und

$X^4$ für Chlor, Brom oder Iod steht,

in Gegenwart eines Verdünnungsmittels umsetzt und in einer zweiten Stufe die so erhaltenen Halogenalkine der Formel

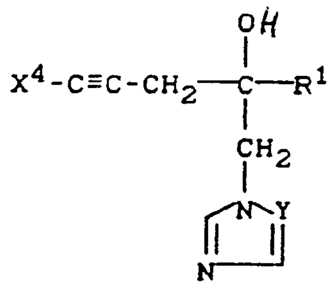

(VI)

in welcher

$R^1$, $X^4$ und Y die oben angegebene Bedeutung haben,

mit Halogen oder Halogen liefernden Verbindungen in Gegenwart eines Verdünnungsmittels umsetzt, und gegebenenfalls anschließend an die so erhaltenen Verbindungen der Formel (I) eine Säure oder ein Metallsalz addiert.

3. Mikrobizide Mittel, gekennzeichnet durch einen Gehalt an mindestens einem Halogenallyl-azolyl-Derivat der Formel (I) gemäß Anspruch 1 bzw. an einem Säureadditions-Salz oder Metallsalz-Komplex eines Halogenallyl-azolyl-Derivates der Formel (I).

4. Verwendung von Halogenallyl-azolyl-Derivaten der Formel (I) gemäß Anspruch 1 bzw. von deren Säureadditions-Salzen und Metallsalz-Komplexen als Mikrobizide im Pflanzenschutz und im Materialschutz.

5. Verfahren zur Bekämpfung von unerwünschten Mikroorganismen im Pflanzenschutz und im Materialschutz, dadurch gekennzeichnet, daß man Halogenallyl-azolyl-Derivate der Formel (I) gemäß Anspruch 1 bzw. deren Säureadditions-Salze oder Metallsalz-Komplexe auf die Mikroorganismen und/oder deren Lebensraum ausbringt.

6. Verfahren zur Herstellung von mikrobiziden Mitteln, dadurch gekennzeichnet, daß man Halogenallyl-azolyl-Derivate der Formel (1) gemäß Anspruch 1 bzw. deren Säureadditions-Salze oder Metallsalz-Komplexe mit Streckmitteln und/oder oberflächenaktiven Stoffen vermischt.

## Claims

1. Halogenoallyl-azolyl derivatives of the formula

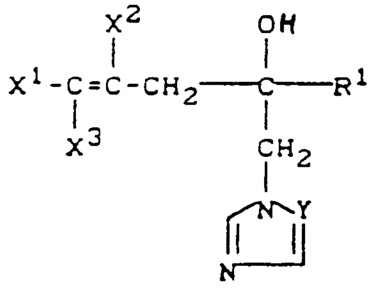

(I)

in which

$R^1$ represents allyl which is monosubstituted to trisubstituted by fluorine, chlorine and/or bromine, or represents propargyl, or represents cycloalkyl which has 3 to 7 carbon atoms, each of these cycloalkyl radicals being monosubstituted to trisubstituted by identical or different substituents, the substituents being alkyl having 1 to 4 carbon atoms and/or halogen, or

$R^1$ represents the radicals of the formulae

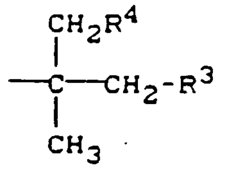

or

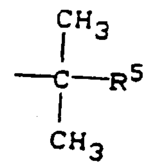

where

$R^3$ represents hydrogen, fluorine, chlorine, bromine, or phenyl which is optionally monosubstituted to trisubstituted by identical or different substituents, the substituents being fluorine, chlorine and/or bromine, or represents phenoxy which is optionally monosubstituted to trisubstituted by identical or different substituents, the substituents being fluorine, chlorine and/or bromine, or represents phenoxymethyl which is optionally monosubstituted to trisubstituted by identical or different substituents, the substituents being fluorine, chorine and/or bromine,

$R^4$ represents hydrogen, fluorine, chlorine or bromine

and

$R^5$ represents iso-propyl, or represents phenyl which is optionally monosubstituted to trisubstituted by identical or different substituents, the substituents being fluorine, chlorine and/or bromine, or represents phenoxy which is optionally monosubstituted to trisubstituted by identical or different substituents, the substituents being fluorine, chlorine and/or bromine,

$X^1$ represents fluorine, chlorine, bromine or iodine,

$X^2$ represents fluorine, chlorine, bromine or iodine,

$X^3$ represents hydrogen, chlorine, bromine or iodine, and

Y represents a nitrogen atom or a CH group,

and their acid addition salts and metal salt complexes.

2. Process for the preparation of halogenoallyl-azolyl derivatives of the formula (I) according to Claim 1 and of their acid addition salts and metal salt complexes, characterised in that

a) alkines of the formula

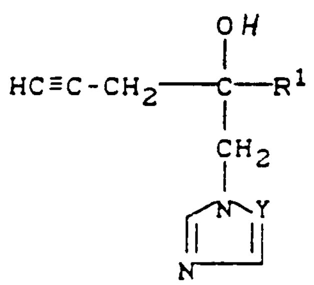

(II)

in which

$R^1$ and Y have the abovementioned meaning,

are reacted with halogen or halogen-donating compounds in the presence of a diluent,

or

b) alkenes of the formula

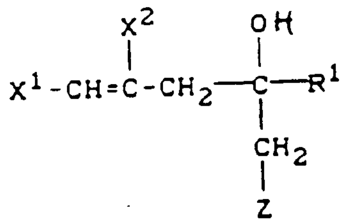

(III)

in which

$R^1$, $X^1$ and $X^2$ have the abovementioned meaning and

Z represents chlorine, bromine, iodine, methylsulphonate or p-tolylsulphonate,

are reacted with azoles of the formula

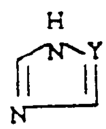

(IV)

in which

Y has the abovementioned meaning,

in the presence of an acid-binding agent and in the presence of a diluent,

or

c) alkines of the formula

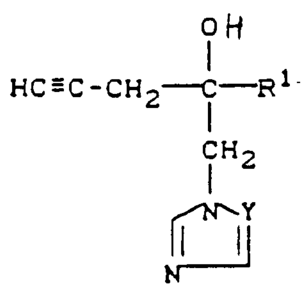

(II)

in which

$R^1$ and Y have the abovementioned meaning,

are reacted, in a first step, with hypohalites of the formula

$MOX^4$ (V)

in which

M represents a sodium or potassium ion and

$X^4$ represents chlorine, bromine or iodine,

in the presence of a diluent, and, in a second step, the resulting halogenoalkines of the formula

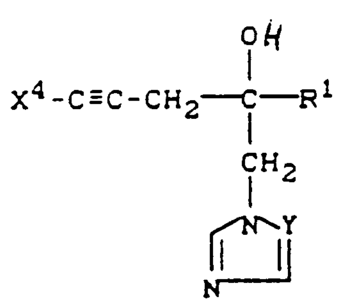

(VI)

in which

$R^1$, $X^4$ and Y have the abovementioned meaning,

are reacted with halogen or halogen-donating compounds in the presence of a diluent,

and, if appropriate, an acid or a metal salt is subsequently added on to the resulting compounds of the formula (I).

**3.** Microbicidal agents, characterised in that they contain at least one halogenoallyl-azolyl derivative of the formula (I) according to Claim 1, or an acid addition salt or metal salt complex of a halogenoallyl-azolyl derivative of the formula (I).

**4.** Use of halogenoallyl-azolyl derivatives of the formula (1) according to Claim 1 or of their acid addition salts and metal salt complexes as microbicides in plant protection and in the protection of materials.

**5.** Method of combating undesirable microorganisms in plant protection and in the protection of materials, characterised in that halogenoallyl-azolyl derivatives of the formula (1) according to Claim 1 or their acid addition salts or metal salt complexes are applied to the microorganisms and/or their environment.

**6.** Process for the preparation of microbicidal agents, characterised in that halogenoallyl-azolyl derivatives of the formula (I) according to Claim 1 or their acid addition salts or metal salt complexes are mixed with extenders and/or surface-active substances.

## Revendications

**1.** Dérivés d'halogénallyl-azolyle de formule

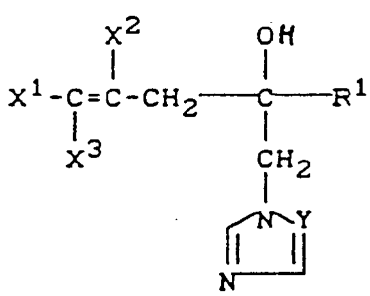

(I)

dans laquelle

$R^1$ est un groupe allyle portant 1 à 3 substituants fluor, chlore et/ou brome, un groupe propargyle ou un groupe cycloalkyle ayant 3 à 7 atomes de carbone, chacun de ces restes cycloalkyle portant 1 à 3 substituants, identiques ou différents, alkyle ayant 1 à 4 atomes de carbone et/ou halogéno, ou bien

$R^1$ représente les restes de formules

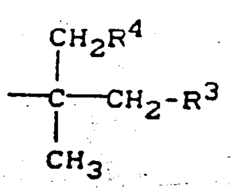

ou

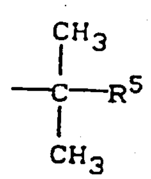

dans lesquelles

$R^3$ est l'hydrogène, le fluor, le chlore, le brome, un groupe phényle portant éventuellement 1 à 3 substituants fluor, chlore et/ou brome identiques ou différentes, un groupe phénoxy portant le cas échéant 1 à 3 substituants fluor, chlore et/ou brome identiques ou différentes ou un groupe phénoxyméthyle portant éventuellement 1 à 3 substituants fluor, chlore et/ou brome identiques ou différents,

$R^4$ représente l'hydrogène, le fluor, le chlore ou le brome

et

$R^5$ est un groupe isopropyle, un groupe phényle portant le cas échéant 1 à 3 substituants fluor, chlore et/ou brome identiques ou différents ou un groupe phénoxy portant le cas échéant 1 à 3 substituants fluor, chlore et/ou brome identiques ou différents,

$X^1$ représente le fluor, le chlore, le brome ou l'iode,

$X^2$ est le fluor, le chlore, le brome ou l'iode,

$X^3$ est l'hydrogène, le chlore, le brome ou l'iode et

Y est un atome d'azote ou un groupe CH,

ainsi que leurs sels d'addition d'acides et leurs complexes de sels métalliques.

**2.** Procédé de production de dérivés d'halogénallyl-azolyle de formule (I) suivant la revendication 1, ainsi que de leurs sels d'addition d'acides et de leurs complexes de sels métalliques caractérisé en ce que

a) on fait réagir des alcynes de formule

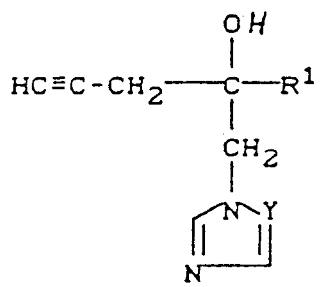

(II)

dans laquelle

$R^1$ et Y ont la définition indiquée ci-dessus,

avec un halogène ou des composés fournissant un halogène en présence d'un diluant,

ou bien

b) on fait réagir des alcènes de formule

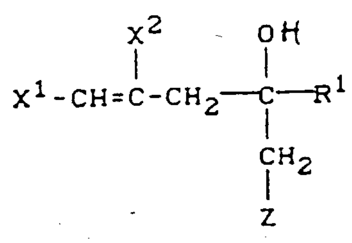

(III)

dans laquelle

$R^1$, $X^1$ et $X^2$ ont la définition indiquée ci-dessus et

Z représente le chlore, le brome, l'iode, un groupe méthylsulfonate ou p-tolylsulfonate,

avec des azoles de formule

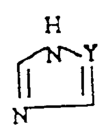

(IV)

dans laquelle

Y a la définition indiquée ci-dessus,

en présence d'un accepteur d'acide et en présence d'un diluant,

ou bien

c) on fait réagir des alcynes de formule

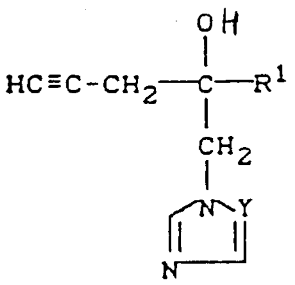

(II)

dans laquelle
$R^1$ et Y ont la définition indiquée ci-dessus,
dans une première étape, avec des hypohalogénites de formule

$$MOX^4 \quad (V)$$

dans laquelle
M est un ion sodium ou potassium et
$X^4$ représente le chlore, le brome ou l'iode,
en présence d'un diluant, et on fait réagir dans une seconde étape les halogénalcynes ainsi obtenus de formule

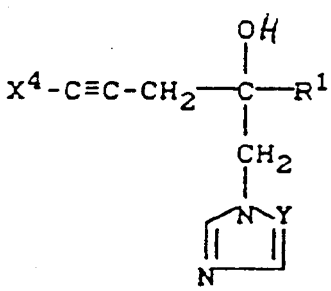

(VI)

dans laquelle
$R^1$, $X^4$ et Y ont la définition indiquée ci-dessus,
avec un halogène ou des composés fournissant un halogène en présence d'un diluant,
puis, le cas échéant, on additionne un acide ou un sel métallique sur les composés de formule (I) ainsi obtenus.

**3.** Compositions microbicides, caractérisées par une teneur en au moins un dérivé d'halogénallyl-azolyle de formule (I) suivant la revendication 1 ou d'un sel d'addition d'acide ou d'un complexe de sel métallique d'un dérivé d'halogénallyl-azolyle de formule (I).

**4.** Utilisation de dérivés d'halogénallyl-azolyle de formule (I) suivant la revendication 1 ou de leurs sels d'addition d'acides et de leurs complexes de sels métalliques comme microbicides dans la protection des plantes et la protection des denrées.

**5.** Procédé pour combattre des micro-organismes indésirables dans la protection des plantes et dans la protection des denrées, caractérisé en ce qu'on fait agir des dérivés d'halogénallyl-azolyle de formule (I) suivant la revendication 1 ou leurs sels d'addition d'acides ou leurs complexes de sels métalliques sur les micro-organismes et/ou sur leur milieu.

**6.** Procédé de préparation de compositions microbicides, caractérisé en ce qu'on mélange des dérivés d'halogénallyl-azolyle de formule (I) suivant la revendication 1, ou leurs sels d'addition d'acides ou leurs complexes de sels métalliques avec des diluants et/ou des agents tensio-actifs.